# FASCICULE DE BREVET EUROPEEN

(11) **EP 4 057 890 B1**
(45) Date de publication et mention de la délivrance du brevet: **31.12.2025**
(21) Numéro de dépôt: 20823893.1
(22) Date de dépôt: 13.11.2020
(51) Int. Cl.: A61B 5/00, A61B 5/024, A61B 5/145

(54) **SYSTÈME DE SURVEILLANCE CORPORELLE**
KÖRPERÜBERWACHUNGSSYSTEM
BODY MONITORING SYSTEM

(30) Priorité: 15.11.2019 FR 1912801
(43) Date de publication de la demande: 21.09.2022
(73) Titulaire: WIZP AS, 3125 Tønsberg (NO)
(72) Inventeur: LE, Anh-minh, 92200 NEUILLY SUR SEINE (FR); PIERART, Luc, 94800 VILLEJUIF (FR); BADIOLA, Florian, 75006 PARIS (FR)
(74) Mandataire: Regimbeau
(86) Numéro de dépôt international: PCT/FR2020/052084
(87) Numéro de publication internationale: WO 2021/094699

(56) Documents cités:
- CN-A- 108 310 615
- US-A1- 2015 173 631
- US-A1- 2018 184 979
- US-A1- 2019 254 602

## Description

### DOMAINE DE L'INVENTION

La présente invention concerne un dispositif portable de mesure d'une grandeur physiologique du corps humain, tel qu'un bracelet ou une montre par exemple.

### ETAT DE LA TECHNIQUE

L'intérêt des consommateurs pour la santé personnelle a conduit à la mise sur le marché d'une variété d'appareils de surveillance de la santé personnelle. Il est par exemple connu de mesurer la fréquence cardiaque avec un dispositif portable attaché au corps d'un utilisateur, par exemple une montre. La montre présente une face destinée à venir en contact avec la peau de l'utilisateur. Cette face présente de manière connue un bossage, présentant une paroi munie d'un capteur de fréquence cardiaque. Dans le cas d'un capteur de fréquence cardiaque optique, le bossage permet d'exercer une pression sur la peau de l'utilisateur supérieure à la pression exercée par le reste de la surface en contact avec la peau, de manière à minimiser le bruit de mesure entraîné par la lumière extérieure.

Toutefois, lors de l'utilisation d'un tel dispositif, la partie de la peau sujette à une mesure ne peut pas être choisie avec précision. En effet, elle est imposée par la géométrie de la montre et celle du poignet de l'utilisateur, après avoir attaché la montre au poignet.

De plus, des mouvements d'une trop grande amplitude peuvent entraîner une rupture du contact entre le bossage et la peau de l'utilisateur, et augmenter le bruit de mesure lié à la lumière environnante.

Enfin, il est désirable de pouvoir coupler la mesure de la fréquence cardiaque avec d'autres mesures de grandeur physiologique du corps humain. Toutefois, le bossage présente une superficie sur laquelle seulement un nombre limité de capteurs peuvent être intégrés. US 2019/0254602 A1 décrit un dispositif comprenant un boîtier de capteur optique associé à un élément de fixation conçu pour être appliqué contre la peau du patient. Le boîtier est monté sur un support jetable constitué d'une membrane souple comportant une surface adhésive. Un mécanisme de clips permet le maintien du boîtier sur le support tout en autorisant sa rotation.

CN 108310615 A concerne un dispositif intégrant un module de détection physiologique des microaiguilles photosensibles. Le système comprend un support porté sur la peau, de type bracelet, équipé de capteurs électrochimiques permettant de mesurer diverses grandeurs physiologiques telles que la température, le glucose ou les ions présents dans la sueur.

### EXPOSE DE L'INVENTION

Un but de l'invention est de proposer une solution pour fabriquer un système permettant de répondre au moins en partie aux problèmes posés par l'art antérieur.

Ce but est atteint dans le cadre de la présente invention grâce à un dispositif de surveillance corporelle, comprenant :
- un boîtier, et
- un système de support comprenant :
   une première face adaptée à être mise en contact avec la peau d'un utilisateur, un patch comprenant une couche d'adhésif, la couche d'adhésif étant agencée sur la première face,
   une capsule configurée pour s'engager avec le patch et/ou avec le boîtier de manière amovible,
   la capsule comprenant :
      - une ou plusieurs microaiguille(s) agencées sur la première face,
      - plusieurs microélectrodes portées en partie par la ou les microaiguilles, la capsule étant configurée pour transmettre des informations représentatives du potentiel des microélectrodes au boîtier,
   dans lequel :
      - le boîtier (5) comprend un module de mesure adapté à mesurer au moins une grandeur physiologique du corps humain,
      - le boîtier comprend un bossage, le module présentant une ou plusieurs paroi(s) agencée(s) sur le bossage,
      - la capsule comprend une cavité, la cavité présentant au moins une première ouverture sur une deuxième face du système de support opposée à première face par rapport au système de support,
      - la cavité présente une forme adaptée à recevoir le bossage dans la cavité,
      - la cavité comprend une deuxième ouverture sur la première face, ou, le module comprend un émetteur de lumière et un détecteur de lumière et le système de support comprend un ou des matériau(x) séparant la cavité de la première face, le ou les matériau(x) étant transparent(s) à la lumière émise par le module.

L'invention est avantageusement complétée par les caractéristiques suivantes, prises individuellement ou en l'une quelconque de leurs combinaisons techniquement possibles :
- le module de mesure est un module de mesure optique adapté à mesurer optiquement au moins une grandeur physiologique du corps humain, le module comprenant un émetteur de lumière et un détecteur de lumière, et l'émetteur de lumière et le détecteur de lumière présentent chacun une des parois agencées sur le bossage,
- la cavité comprend une deuxième ouverture sur la première face, et le module de mesure est un module de mesure électrique, comprenant au moins deux électrodes agencées en surface du bossage,
- la grandeur physiologique du corps humain est la fréquence cardiaque,
- le dispositif comprend des moyens d'attache au corps de l'utilisateur adaptés à ce que le boîtier exerce une pression sur le système de support lors de l'attache du dispositif au corps de l'utilisateur,
- la couche d'adhésif est transparente et recouvre la cavité,
- la capsule comprend des moyens de transmission d'une information représentative d'un élément au moins choisi parmi la concentration d'un analyte dans le corps de l'utilisateur, la température et des identifiants de la capsule,
- chaque microélectrode présente une connexion électrique sur la deuxième face du système de support opposée à première face par rapport au système de support, le boîtier comprenant des connexions électriques agencées autour du bossage et configurées pour être en contact avec les connexions électriques de la capsule, préférentiellement lorsque la capsule est engagée avec le boîtier,
- la forme du bossage et la forme de la cavité sont configurées pour que au moins la moitié du volume du bossage soit insérée dans la cavité lorsque la capsule est engagée avec le boîtier,
- les moyens d'attache au corps sont choisis au moins parmi un bracelet configuré pour entourer un membre de l'utilisateur et préférentiellement un bracelet-montre, une sangle, les moyens d'attache au corps étant réutilisables,
- la forme de la cavité et la forme du bossage sont configurées pour n'autoriser qu'un nombre de position du boîtier par rapport au système de support inférieur à 20, et préférentiellement inférieur à 4, lorsque la capsule est engagée avec le boîtier,
- le dispositif comprend une partie configurée pour former joint entre la cavité et la première face lorsque la première face est en contact avec la peau d'un utilisateur.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
[Fig. 1] La figure 1 illustre schématiquement une coupe d'un dispositif de surveillance corporelle conforme à l'invention.
[Fig. 2] La figure 2 illustre schématiquement un dispositif de surveillance corporelle conforme à l'invention, en vue éclatée, de dessous.
[Fig. 3] La figure 3 illustre schématiquement un dispositif de surveillance corporelle conforme à l'invention, dans lequel le bossage est reçu par la cavité.
[Fig. 4] La figure 4 illustre schématiquement un dispositif de surveillance corporelle conforme à l'invention, en vue éclatée, dans lequel le boîtier et le système de support sont séparés.

Sur l'ensemble des figures, les éléments similaires portent des références identiques.

### DEFINITIONS

On entend par « microélectrode » une électrode comprenant au moins une partie, désignée par « partie active », configurée pour être introduite dans la peau d'un utilisateur, la longueur de cette partie étant comprise entre 1 µm et 999 µm. Par ailleurs, la microélectrode peut comprendre une autre partie d'une longueur supérieure.

On entend par « transparent » tout matériau qui laisse passer la lumière et qui ne fait pas écran au discernement d'un objet dont le module optique permet de faire l'image. Un matériau transparent peut être en partie opaque. Dans les modes de réalisation de l'invention, la couche de matériau agencée entre la cavité et la première face, si elle existe, est configurée pour qu'au moins 50 % de l'énergie lumineuse soit transmise au travers de la couche.

### DESCRIPTION DETAILLEE DE L'INVENTION

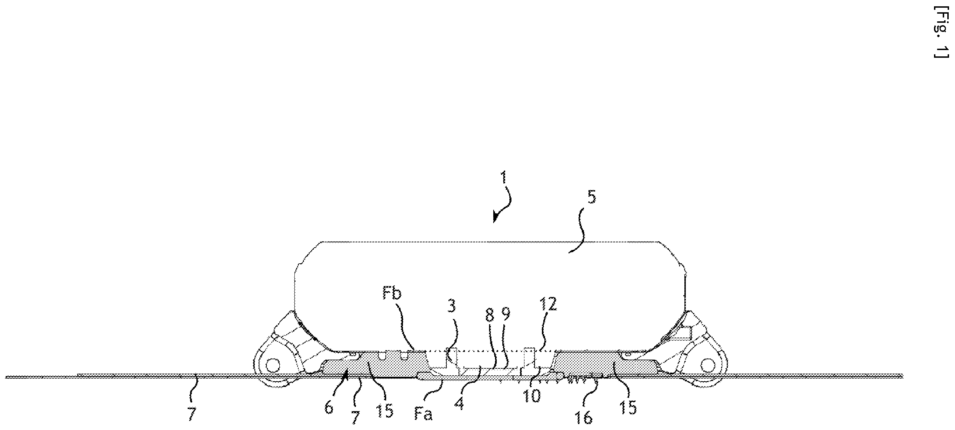

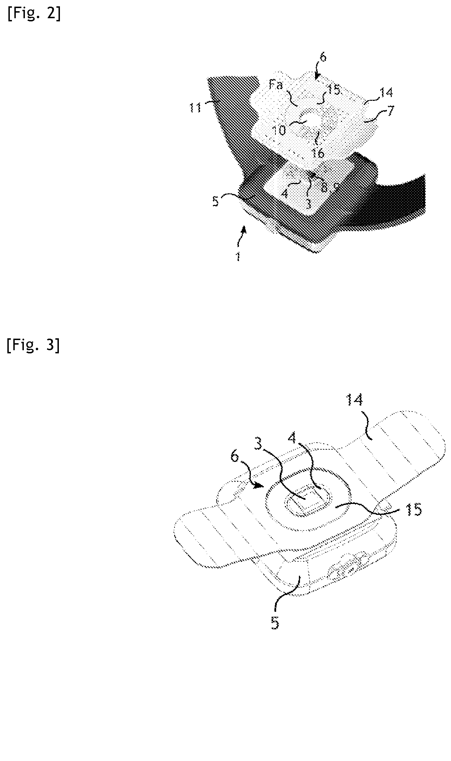

En référence à la figure 1 et à la figure 2, un dispositif 1 de surveillance corporelle conforme à l'invention comprend un boîtier 5 et un système de support 6.

Le boîtier 5 comprend préférentiellement une unité de contrôle, l'unité de contrôle comprenant un processeur et un module de mémoire. Le boîtier 5 comprend préférentiellement par exemple des moyens d'affichage, tel qu'un écran LED permettant à l'utilisateur de prendre connaissance des résultats de la surveillance corporelle mise en œuvre par le dispositif 1.

Le boîtier 5 comprend préférentiellement des moyens d'attache 11 au corps, permettant au boîtier 5 d'exercer une pression vers un membre de l'utilisateur, préférentiellement sur un système de support 6 configuré pour adhérer sur la peau de l'utilisateur. Préférentiellement, le bracelet est un bracelet montre et/ou une sangle. Préférentiellement, les moyens d'attache 11 au corps sont réutilisables. Ainsi, le boîtier 5 peut être mis en contact contre la peau de manière réversible, à volonté.

Le boîtier 5 comprend un bossage 4 sur l'une de ses faces. Le boîtier 5 comprend un module de mesure 3 adapté à mesurer au moins une grandeur physiologique du corps humain, le module 3 état logé au moins en partie dans le bossage 4. La grandeur physiologique peut être préférentiellement choisie au moins parmi la fréquence cardiaque, la tension artérielle, une mesure du stress de l'utilisateur, une mesure de la proportion de graisses dans une partie du corps de l'utilisateur et/ou une mesure de la masse musculaire dans une partie du corps de l'utilisateur. Le module 3 de mesure présente au moins une paroi, agencée sur le bossage 4 du boîtier 5.

Le module 3 de mesure est préférentiellement un module 3 de mesure optique, adapté à mesurer optiquement au moins grandeur physiologique du corps humain. Le module 3 peut comprendre un émetteur 8 de lumière et un détecteur 9 de lumière, l'émetteur 8 de lumière et le détecteur 9 de lumière présentant chacun une paroi agencée sur le bossage 4. L'émetteur 8 peut préférentiellement comprendre une LED, configurée pour émettre de la lumière au travers de la paroi puis au travers de la peau d'un utilisateur, le détecteur 9 étant configuré pour mesurer la diffraction de la lumière émise par l'émetteur 8 par les vaisseaux sanguins agencés sous la peau de l'utilisateur. En plus de mesurer le rythme cardiaque, le module 3 est préférentiellement configuré pour mesurer la saturation en oxygène dans le sang, également appelé SpO₂. Préférentiellement, le module 3 est configuré pour établir un photopléthysmogramme de l'utilisateur.

Le dispositif 1 comprend également un système de support 6. Le système de support 6 comprend une première face Fa adaptée à être mise en contact avec la peau de l'utilisateur. Préférentiellement, le système de support 6 comprend une couche 7 d'adhésif agencée sur la première face Fa.

Le système de support 6 comprend préférentiellement un patch 14 et une capsule 15. Le patch 14 est configuré pour adhérer à la peau, par exemple au moyen de la couche 7 d'adhésif. La capsule 15 est configurée pour s'engager avec le patch 6 et/ou avec le boîtier 5 de manière amovible. La capsule 15 peut préférentiellement être engagée dans le patch 14 au moyen de points d'arrêt enclenchés par rotation de la capsule 15 par rapport au patch 14. Ainsi, il est possible d'intégrer des systèmes de mesure d'une grandeur physiologique du corps humain au dispositif 1 dans une partie du dispositif 1 dont le mouvement par rapport à la peau est limité, voire nul.

Préférentiellement, l'adhésif peut être choisi de sorte à entraîner une résistance au pelage du patch 6 comprise entre 0,001 N/mm et 1,4 N/mm, notamment comprise entre 0,002 et 0,03 N/mm, et préférentiellement comprise entre 0,01 N/mm et 0,02 N/mm. Ces gammes de résistance correspondent à des adhésions qualifiées de faible à la peau par l'homme du métier. *Lui et al.* (Liu, L., Kuffel, K., Scott, D. K., Constantinescu, G., Chung, H. J., & Rieger, J. (2017), Silicone-based adhesives for long-term skin application: cleaning protocols and their effect on peel strength. Biomedical Physics & Engineering Express, 4(1), 015004) décrivent par exemple des méthodes de mesure de la résistance au pelage d'un patch comprenant un adhésif à la peau. L'adhésif est préférentiellement un élastomère sensible à la pression. Un tel adhésif est par exemple différent des adhésifs de type résine acrylique, qui peuvent lier le patch 6 à la peau avec une force d'adhésion plus élevée. Préférentiellement, l'élastomère sensible à la pression comprend au moins un élément choisi parmi du caoutchouc naturel, du caoutchouc de silicone (dit silicone, ou *«silicone rubber»* en anglais), du caoutchouc d'acrylonitrile, du caoutchouc de polyuréthane, des polyisobutylènes de bas poids moléculaire et un gel de silicone. Préférentiellement, l'élastomère sensible à la pression comprend du caoutchouc de silicone (ou simplement silicone). L'adhésif peut être également un adhésif à base d'acrylique, dans lequel la concentration surfacique d'acrylique est adaptée pour que la résistance de pelage du patch à la peau soit dans l'une des gammes précitées. Ainsi, il est possible d'enlever le patch 6 de la peau et de le coller de nouveau sur la peau si la première application du patch 6 est mise en œuvre à un endroit qui ne convient pas à l'utilisateur. Le retrait du patch 6 peut être mis en œuvre sans arrachage de poil et/ou sans irritation. De plus, des allergies peuvent être évitées à l'utilisateur.

Le système de support 6, en particulier la capsule 15, comprend une cavité 10. La cavité 10 présente au moins une première ouverture 12 sur une deuxième face Fb du système de support 6 opposée à la première face Fa par rapport au système de support 6. La cavité 10 présente une forme adaptée à recevoir le bossage 6 dans la cavité 10. La forme de la cavité 10 est préférentiellement complémentaire à la forme du bossage 6. Le bossage 6 peut préférentiellement être agencé au moins en majorité de volume à l'intérieur de la cavité 10, de par la forme de la cavité 10 et la forme du bossage 6.

En référence à la figure 1, le bossage 4 peut être de forme tronconique. Dans d'autres modes de réalisation de l'invention, la forme du bossage 4 peut être cylindrique, hémisphérique, et/ou présenter un axe d'extension selon une direction parallèle à la première face Fa.

La cavité 10 comprend une deuxième ouverture 13 sur la première face Fa, ou bien un ou des matériaux du système de support 6 séparant la cavité 10 de la première face Fa sont transparents à une lumière émise par le module 3.

Cet agencement permet plusieurs effets concomitants : la position à laquelle le module 3 est amené près de la peau ou au contact de la peau peut être choisie de manière précise de par la disposition du patch 14 sur la peau de l'utilisateur. De plus, la cavité 10 permet de protéger le module 3 des interférences ou du bruit qui pourraient être exercés lors de la mesure de la grandeur physiologique. Notamment, la cavité 10 empêche la lumière extérieure d'accéder jusqu'au détecteur 9 de lumière. De plus, le système de support 6 permet de bloquer d'éventuels mouvements de la montre lors de la mesure de la grandeur physiologique, en particulier lors de la mesure du rythme cardiaque.

Lorsque la cavité 10 comprend une deuxième ouverture 13 sur la première face Fa, la peau au contact de la première face Fa peut légèrement être bombée à l'intérieur la cavité 10, de par sa souplesse. Ainsi, il est possible de contrôler avec exactitude la localisation de la peau par rapport au dispositif 1 de sorte à améliorer la mesure de la grandeur physiologique.

La deuxième ouverture 13 et le ou les matériaux transparents permettent au module 3 de mesurer la grandeur physiologique au travers de la peau, par exemple optiquement et ou électriquement.

La couche 7 d'adhésif peut préférentiellement être transparente et recouvrir la cavité 10. De cette manière, la couche 7 d'adhésif sépare la cavité 10 de l'extérieur du dispositif 1, tout en permettant à un module 3 optique de mesurer la fréquence cardiaque en utilisant une lumière passant au travers de la couche 7 d'adhésif.

Le dispositif 1 comprend préférentiellement une partie configurée pour former joint entre la cavité 10 et la première face Fa lorsque la première face Fa est en contact avec la peau d'un utilisateur. Ainsi, si un liquide, par exemple de l'eau, parvient à entrer dans la cavité 10 par la première ouverture 12, le liquide ne peut pas s'introduire entre la peau de l'utilisateur et la première face Fa. Ainsi, le développement de bactéries lors de l'utilisation du dispositif 1 est évitée. La partie configurée pour former joint peut préférentiellement être fabriquée dans un matériau semi-perméable, en particulier perméable à l'air et imperméable au liquide.

La partie configurée pour former joint présente préférentiellement une forme de révolution et s'étend préférentiellement entre la paroi de la cavité 10 et la paroi du bossage 4.

En variante ou en combinaison, la partie configurée pour former joint est disposée le long de l'arête formée par l'intersection de la cavité 10 et de la première face Fa.

En variante ou en combinaison, la partie configurée pour former joint comprend la couche 7 d'adhésif recouvrant la cavité 10.

Le dispositif 1 peut également comprendre plusieurs paires bossage 4/cavité 10, chaque bossage 4 étant présenté par le boîtier 5, et chaque cavité 10 étant présentée par le système de support 5, et préférentiellement par la capsule 15.

La cavité 10 présente préférentiellement une forme complémentaire à une forme tronconique du bossage, ou une forme cylindrique. La hauteur de la cavité 10, c'est-à-dire la taille de la cavité 10 dans la direction perpendiculaire à la première face Fa, est préférentiellement comprise entre 500 µm et 1,5 cm, et notamment comprise entre 1 mm et 1 cm. La hauteur du bossage 4, c'est-à-dire la taille du bossage 4 dans la direction perpendiculaire à la première face Fa, est préférentiellement comprise entre 500 µm et 1,5 cm, et notamment comprise entre 1 mm et 1 cm.

La capsule 15 comprend une ou plusieurs microaiguilles 16 agencées sur la première face Fa. La capsule 15 comprend alors plusieurs microélectrodes portées en partie par la ou les microaiguilles. La capsule 15 est configurée pour transmettre des informations représentatives du potentiel électrique des microélectrodes au boîtier 5. La taille et la forme des microaiguilles 16 sont configurées pour pénétrer dans la peau de l'utilisateur, au moins jusqu'à une couche dans laquelle le liquide interstitiel est présent. Ainsi, le dispositif 1 peut être configuré pour mesurer plusieurs grandeurs physiologiques différentes du corps humain. Préférentiellement, le dispositif 1 est configuré pour mesurer la concentration d'un analyte dans un liquide corporel, et préférentiellement pour mesurer la concentration du glucose et/ou du lactate dans le liquide interstitiel.

La transmission des informations mesurées par le dispositif 1, préférentiellement représentatives d'une concentration d'analyte dans le liquide corporel, de la température, et/ou d'un identifiant électronique de la capsule 15, peut être électrique et/ou radio.

La capsule 15 peut préférentiellement comprendre un émetteur et/ou récepteur radio, et, le boîtier 5 peut respectivement comprendre un récepteur et/ou un émetteur radio. D'autres moyens de communication sans fil connus peuvent être utilisés.

Chaque microélectrode présente préférentiellement une connexion électrique sur la deuxième face Fb du système de support 6 opposée à première face Fa par rapport au système de support 6, le boîtier 5 comprenant des connexions électriques agencées autour du bossage 4 et configurées pour être en contact avec les connexions électriques de la capsule 15. Ainsi, la transmission des informations mesurées par le dispositif 1, représentatives d'une concentration d'analyte dans le liquide corporel, peut être mise en œuvre lorsque la capsule 15 est engagée avec le boîtier 5 de manière amovible.

En référence à la figure 2, le module 3 peut comprendre un système de mesure électrique et/ou par impédance d'une grandeur physiologique de l'utilisateur, préférentiellement du rythme cardiaque de l'utilisateur. Le système de mesure électrique est préférentiellement choisi parmi un système de mesure EEG, PPG et ECG.

Préférentiellement, le bossage 4 présente une face plane. Lorsque le bossage 4 est de forme tronconique, la face plane correspond au sommet de la forme tronconique. La ou les interfaces des différents capteurs du module 3 sont préférentiellement agencés sur la face plane. En particulier, la figure 2 illustre la paroi d'un émetteur 8 de lumière et d'un détecteur 9 de lumière au centre de la face plane, ainsi que des électrodes disposées de part et d'autre de la paroi. Ces électrodes peuvent être reliées au boîtier 5 et configurées pour mettre en œuvre une mesure électrique et/ou par impédance de la grandeur physiologique, et préférentiellement du rythme cardiaque. Les électrodes peuvent être agencées sur la surface du bossage, notamment autour du module 3 de mesure optique.

En référence à la figure 3, le patch 6 et le boîtier 5 peuvent être engagés de manière amovible. La figure 3 illustre un dispositif 1 dans lequel la cavité 10 présente une deuxième ouverture 13 de sorte à permettre un contact entre le module 3 et la peau.

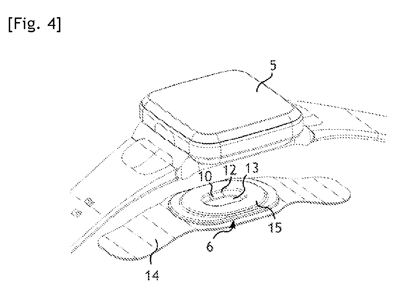

En référence à la figure 4, le patch 6 et la capsule 15 peuvent être engagés avant l'application du système de support 6 sur la peau. Après l'adhésion du système de support 6 sur la peau, le boîtier 5 peut être engagé avec le système de support 6, en particulier avec la capsule 15.

## Revendications

1. Dispositif (1) de surveillance corporelle, comprenant :
- un boîtier (5), et
- un système de support (6) comprenant :
une première face (Fa) adaptée à être mise en contact avec la peau d'un utilisateur,
un patch (14) comprenant une couche d'adhésif (7), la couche d'adhésif (7) étant agencée sur la première face (Fa),
une capsule (15) configurée pour s'engager avec le patch (6) et/ou avec le boîtier (5) de manière amovible,
la capsule (15) comprenant :
- une ou plusieurs microaiguille(s) (16) agencées sur la première face (Fa),
- plusieurs microélectrodes portées en partie par la ou les microaiguilles (16), la capsule (15) étant configurée pour transmettre des informations représentatives du potentiel des microélectrodes au boîtier (5),
le dispositif (1) possédant également les caractéristiques suivantes :
- le boîtier (5) comprend un module (3) de mesure adapté à mesurer au moins une grandeur physiologique du corps humain,
- le boîtier (5) comprend un bossage (4), le module (3) présentant une ou plusieurs paroi(s) agencée(s) sur le bossage (4),
- la capsule comprend une cavité (10), la cavité (10) présentant au moins une première ouverture (12) sur une deuxième face (Fb) du système de support (6) opposée à première face (Fa) par rapport au système de support (6),
- la cavité (10) présente une forme adaptée à recevoir le bossage (4) dans la cavité (10),
- la cavité (10) comprend une deuxième ouverture (13) sur la première face (Fa), ou, le module (3) comprend un émetteur (8) de lumière et un détecteur (9) de lumière et le système de support (6) comprend un ou des matériau(x) séparant la cavité (10) de la première face (Fa), le ou les matériau(x) étant transparent(s) à la lumière émise par le module (3).

2. Dispositif (1) conforme à la revendication 1, dans lequel le module (3) de mesure est un module (3) de mesure optique adapté à mesurer optiquement au moins une grandeur physiologique du corps humain, le module (3) comprenant un émetteur (8) de lumière et un détecteur (9) de lumière, et l'émetteur (8) de lumière et le détecteur de lumière (9) présentent chacun une des parois agencées sur le bossage (4).

3. Dispositif (1) conforme à la revendication 1 ou 2, comprenant des moyens d'attache (11) au corps de l'utilisateur adaptés à ce que le boîtier (5) exerce une pression sur le système de support (6) lors de l'attache du dispositif (1) au corps de l'utilisateur.

4. Dispositif (1) conforme à l'une des revendications 1 à 3, dans lequel la couche d'adhésif (7) est transparente et recouvre la cavité (10).

5. Dispositif (1) conforme à l'une des revendications 1 à 4, dans lequel chaque microélectrode présente une connexion électrique sur la deuxième face (Fb) du système de support (6) opposée à première face (Fa) par rapport au système de support (6), le boîtier (5) comprenant des connexions électriques agencées autour du bossage (4) et configurées pour être en contact avec les connexions électriques de la capsule (15).

6. Dispositif (1) conforme à l'une des revendications 1 à 5, dans lequel la forme du bossage (4) et la forme de la cavité (10) sont configurées pour que au moins la moitié du volume du bossage soit inséré dans la cavité (10) lorsque la capsule (15) est engagée avec le boîtier (5).

7. Dispositif (1) conforme à l'une des revendications 1 à 6, dans lequel la forme de la cavité (10) et la forme du bossage (4) sont configurées pour n'autoriser qu'un nombre de position du boîtier (5) par rapport au système de support (6) inférieur à 20, et préférentiellement inférieur à 4, lorsque la capsule (15) est engagée avec le boîtier (5).

8. Dispositif (1) conforme à l'une des revendications 1 à 7, comprenant une partie configurée pour former joint entre la cavité (10) et la première face (Fa) lorsque la première face (Fa) est en contact avec la peau d'un utilisateur.

## Patentansprüche

1. Körperüberwachungsvorrichtung (1), umfassend:
- ein Gehäuse (5), und
- ein Trägersystem (6), umfassend:
eine erste Seite (Fa), die geeignet ist, mit der Haut eines Benutzers in Kontakt zu kommen,
ein Patch (14), das eine Haftschicht (7) umfasst, wobei die Haftschicht (7) auf der ersten Seite (Fa) eingerichtet ist,
eine Kapsel (15), die ausgelegt ist, um lösbar mit dem Patch (6) und/oder dem Gehäuse (5) in Eingriff gebracht zu werden,
wobei die Kapsel (15) umfasst:
- eine oder mehrere Mikronadeln (16), die auf der ersten Seite (Fa) eingerichtet sind,
- mehrere Mikroelektroden, die teilweise von der oder den Mikronadeln (16) getragen werden, wobei die Kapsel (15) ausgelegt ist, um Informationen, die für das Potenzial der Mikroelektroden repräsentativ sind, an das Gehäuse (5) zu übertragen,
wobei die Vorrichtung (1) ebenfalls die folgenden Merkmale besitzt:
- das Gehäuse (5) umfasst ein Messmodul (3), das geeignet ist, mindestens eine physiologische Größe des menschlichen Körpers zu messen,
- das Gehäuse (5) umfasst eine Erhebung (4), wobei das Modul (3) eine oder mehrere Wände aufweist, die auf der Erhebung (4) eingerichtet sind,
- die Kapsel umfasst einen Hohlraum (10), wobei der Hohlraum (10) mindestens eine erste Öffnung (12) auf einer zweiten Seite (Fb) des Trägersystems (6) aufweist, die der ersten Seite (Fa) in Bezug auf das Trägersystem (6) gegenüberliegt,
- der Hohlraum (10) weist eine Form auf, die zur Aufnahme der Erhebung (4) in dem Hohlraum (10) geeignet ist,
- der Hohlraum (10) umfasst eine zweite Öffnung (13) auf der ersten Seite (Fa), oder das Modul (3) umfasst einen Lichtsender (8) und einen Lichtdetektor (9) und das Trägersystem (6) umfasst ein oder mehrere Materialien, die den Hohlraum (10) von der ersten Seite (Fa) trennen, wobei das oder die Materialien für das vom Modul (3) gesendet Licht transparent ist/sind.

2. Vorrichtung (1) nach Anspruch 1, wobei das Messmodul (3) ein optisches Messmodul (3) ist, das geeignet ist, mindestens eine physiologische Größe des menschlichen Körpers optisch zu messen, wobei das Modul (3) einen Lichtsender (8) und einen Lichtdetektor (9) umfasst und der Lichtsender (8) und der Lichtdetektor (9) jeweils eine der an der Erhebung (4) angeordneten Wände aufweisen.

3. Vorrichtung (1) nach Anspruch 1 oder 2, umfassend Befestigungsmittel (11) am Körper des Benutzers, die dazu geeignet sind, dass das Gehäuse (5) beim Befestigen der Vorrichtung (1) am Körper des Benutzers einen Druck auf das Trägersystem (6) ausübt.

4. Vorrichtung (1) nach einem der Ansprüche 1 bis 3, wobei die Haftschicht (7) transparent ist und den Hohlraum (10) bedeckt.

5. Vorrichtung (1) nach einem der Ansprüche 1 bis 4, wobei jede Mikroelektrode einen elektrischen Anschluss auf der zweiten Seite (Fb) des Trägersystems (6) aufweist, die der ersten Seite (Fa) in Bezug auf das Trägersystem (6) gegenüberliegt, wobei das Gehäuse (5) elektrische Anschlüsse umfasst, die um die Erhebung (4) herum eingerichtet und ausgelegt sind, um mit den elektrischen Anschlüssen der Kapsel (15) in Kontakt zu stehen.

6. Vorrichtung (1) nach einem der Ansprüche 1 bis 5, wobei die Form der Erhebung (4) und die Form des Hohlraums (10) ausgelegt sind, damit mindestens die Hälfte des Volumens der Erhebung in den Hohlraum (10) eingeführt wird, wenn die Kapsel (15) mit dem Gehäuse (5) in Eingriff steht.

7. Vorrichtung (1) nach einem der Ansprüche 1 bis 6, wobei die Form des Hohlraums (10) und die Form der Erhebung (4) ausgelegt sind, um nur eine Anzahl von Positionen des Gehäuses (5) weniger als 20 und vorzugsweise weniger als 4 im Verhältnis zum Trägersystem (6) zu gestatten, wenn die Kapsel (15) mit dem Gehäuse (5) in Eingriff steht.

8. Vorrichtung (1) nach einem der Ansprüche 1 bis 7, umfassend einen Teil, der ausgelegt ist, um eine Dichtung zwischen dem Hohlraum (10) und der ersten Seite (Fa) zu bilden, wenn die erste Seite (Fa) mit der Haut eines Benutzers in Kontakt steht.

## Claims

1. Body monitoring device (1), comprising:
- a housing (5), and
- a support system (6) comprising:
a first face (Fa) adapted to be brought into contact with the skin of a user,
a patch (14) comprising an adhesive layer (7), the adhesive layer (7) being arranged on the first face (Fa),
a capsule (15) configured to engage with the patch (6) and/or with the housing (5) in a removable manner,
the capsule (15) comprising:
- one or more microneedles (16) arranged on the first face (Fa),
- a plurality of microelectrodes carried in part by the micro-needle(s) (16), the capsule (15) being configured to transmit information representative of the potential of the microelectrodes to the housing (5),
the device (1) also having the following features:
- the housing (5) comprises a measurement module (3) adapted to measure at least one physiological parameter of the human body,
- the housing (5) comprises a boss (4), the module (3) having one or more walls arranged on the boss (4),
- the capsule comprises a cavity (10), the cavity (10) having at least one first opening (12) on a second face (Fb) of the support system (6) opposite the first face (Fa) with respect to the support system (6),
- the cavity (10) has a shape adapted to receive the boss (4) in the cavity (10),
- the cavity (10) comprises a second opening (13) on the first face (Fa), or, the module (3) comprises a light emitter (8) and a light detector (9) and the support system (6) comprises one or more materials separating the cavity (10) from the first face (Fa), the material(s) being transparent to the light emitted by the module (3).

2. Device (1) according to claim 1, wherein the measuring module (3) is an optical measuring module (3) adapted to optically measure at least one physiological parameter of the human body, the optical module (3) comprising a light emitter (8) and a light detector (9), and the light emitter (8) and the light detector (9) each having one of the walls arranged on the boss (4).

3. Device (1) according to claim 1 or 2, comprising means (11) for attaching to the user's body, adapted so that the housing (5) exerts pressure on the support system (6) when the device (1) is attached to the user's body.

4. Device (1) according to any one of claims 1 to 3, wherein the adhesive layer (7) is transparent and covers the cavity (10).

5. Device (1) according to any one of claims 1 to 4, wherein each microelectrode has an electrical connection on the second face (Fb) of the support system (6) opposite the first face (Fa) with respect to the support system (6), the housing (5) comprising electrical connections arranged around the boss (4) and configured to be in contact with the electrical connections of the capsule (15).

6. Device (1) according to any one of claims 1 to 5, wherein the shape of the boss (4) and the shape of the cavity (10) are configured such that at least half of the volume of the boss is inserted into the cavity (10) when the cap (15) is engaged with the housing (5).

7. Device (1) according to any one of claims 1 to 6, wherein the shape of the cavity (10) and the shape of the boss (4) are configured to allow only a number of positions of the housing (5) relative to the support system (6) less than 20, and preferably less than 4, when the capsule (15) is engaged with the housing (5).

8. Device (1) according to any one of claims 1 to 7, comprising a part configured to form a seal between the cavity (10) and the first face (Fa) when the first face (Fa) is in contact with the skin of a user.
